# EUROPÄISCHE PATENTSCHRIFT

(11) **EP 0 885 206 B1**
(45) Veröffentlichungstag und Bekanntmachung des Hinweises auf die Patenterteilung: **12.06.2002**
(21) Anmeldenummer: 97906172.8
(22) Anmeldetag: 05.03.1997
(51) Int. Cl.: C07D 301/12, B01J 27/18, B01J 27/185, C01B 15/029

(54) **EDELMETALLFREIE KATALYSATORZUSAMMENSETZUNG**
CATALYST COMPOSITION FREE FROM NOBLE METALS
COMPOSITION DE CATALYSEUR EXEMPTE DE METAUX PRECIEUX

(30) Priorität: 05.03.1996 DE 19608493
(43) Veröffentlichungstag der Anmeldung: 23.12.1998
(73) Patentinhaber: BASF AKTIENGESELLSCHAFT, 67056 Ludwigshafen (DE)
(72) Erfinder: MÜLLER, Ulrich, D-67434 Neustadt (DE); SCHULZ, Michael, D-67067 Ludwigshafen (DE); MAROSI, Laszlo, D-67063 Ludwigshafen (DE); HARDER, Wolfgang, D-69469 Weinheim (DE)
(74) Vertreter: Kinzebach, Werner, Dr.
(86) Internationale Anmeldenummer: EP9701113
(87) Internationale Veröffentlichungsnummer: WO9732866

(56) Entgegenhaltungen:
- EP-A- 0 100 119
- EP-A- 0 109 273
- GB-A- 2 055 821
- US-A- 4 952 547

## Beschreibung

Die Erfindung betrifft eine edelmetallfreie, feste Katalysatorzusammensetzung, deren Herstellung, deren Verwendung zur Produktion von Wasserstoffperoxid sowie deren Verwendung bei der Epoxidation von Olefinen.

Wasserstoffperoxid findet heutzutage vielfältige Anwendung als sauberes Oxidationsmittel, wie z.B. zum Bleichen von Papier und Zellstoff, zum Entfernen von SO₂ aus Abgasen, in der Elektronikindustrie bei der Halbleiterfertigung, sowie zur Entkeimung, wie beispielsweise der Desodorierung oder der Desinfizierung von Verpackungsmaterial. In der organischen Chemie verwendet man Wasserstoffperoxid insbesondere in Epoxidierungs- und Hydroxylierungsreaktionen, wobei Wasserstoffperoxid auch in situ erzeugt werden kann.

Zur Herstellung von Wasserstoffperoxid wird heute entsprechend dem Stand der Technik weitgehend nach dem Anthrachinon-Verfahren gearbeitet (vgl. Ullmann's Encyclopedia of Industrial Chemistry, 5. Aufl., Bd. A13, S. 443 ff). Der Teilschritt der Hydrogenierung erfolgt dabei üblicherweise in Gegenwart eines Metall-Katalysators, wie z.B. Palladium-Schwarz oder Raney-Nickel. Darüber hinaus sind heterogenkatalytische Herstellungsverfahren beschrieben, bei denen Edelmetalle auf unterschiedlichen Trägern als Katalysator verwendet werden. So wird in der US 5,320,821 Pd/Heteropolysäure als Katalysator verwendet, um Wasserstoffperoxid aus den Elementen herzustellen. Außerdem ist aus der JP 5017106-A die Verwendung von Silica oder Zeolithen mit Platinmetallen und aus der EP 0 537 836 die Verwendung von Zirkonoxiden mit Pd bekannt.

Vielfach müssen dabei jedoch Halogenverbindungen als Promotoren und Stabilisatoren eingesetzt werden, wie dies z.B. in US 5,320,821 beschrieben wird.

In organischen oxidationsreaktionen kann in situ katalytisch gebildetes Wasserstoffperoxid direkt oder in Kombination mit Peroxosauerstoffüberträgern (vgl. G. Goor in G. Strukul, "Catalytic Oxidations with Hydrogen Peroxide as Oxidant", S. 13-43, 1992 Kluwer Academic Publishers) eingesetzt werden. Insbesondere sind als heterogene Oxidationskatalysatoren titanhaltige Zeolithe bekannt, deren Herstellung z.B. in DE 3047798 beschrieben ist. Zeolithe dieses Typs verwendet man, um Sauerstoff auf Mono- und Diolefine zu übertragen (vgl. EP 0 100 119 und EP 0 190 609). Gegenüber der technischen Oxidation nach dem Chlorhydrin-Verfahren (vgl. K. Weissermel, H.-J. Arpe, "Industrielle Organische Chemie", 3. Aufl., VCH Verlag (1988) S. 284-289) besitzt das Verfahren nach EP 0 100 119 den Vorteil,' beispielsweise Propylenoxid aus Propen in hoher Selektivität zugänglich zu machen.

Tatsumi beschreibt in J. Chem. Soc. Chem. Commun. (1992) 1446-7) die Hydroxylierung von Benzol und die Oxidation von Hexan mit Wasserstoff/Sauerstoff an metallischem Palladium auf TS-1 Silikalit, wobei jedoch nur geringe Reaktionsraten verglichen mit wasserstoffperoxid beobachtet wurden.

Die US-A-4,952,547 beschreibt einen Katalysator für die oxidative Kupplung von Methan zu höheren Kohlenwasserstoffen. Der Katalysator wird aus einem wässrigen Gemisch von Eisen(III)nitrat oder Eisen(III)ammoniumnitrat mit Phosphorsäure oder Alkaliphosphat auf einem Träger gebildet. Es wird zunächst eine Katalysatorvorstufe erzeugt, welche z.B. bei 75°C getrocknet wird.

Die EP-A-0 109 273 beschreibt einen Epoxidations-Katalysator bestehend aus einer peroxidischen Wolframverbindung. Die Katalysatorzusammensetzung wird aus einer Wolframverbindung, einer Phosphat- oder Arsenquelle, Wasserstoffperoxid in wässriger Lösung und einem quaternären Oniumsalz gebildet.

Aus der DE-OS 44 25 672 sind außerdem verbesserte titanzeolithhaltige Edelmetall-Katalysatoren und Verfahren zur Herstellung von Propylenoxid aus Wasserstoff, Sauerstoff und Propen bekannt. Die darin beschriebenen Katalysatorsysteme sind z.B. in Bezug auf Reaktivität, Selektivität und Stabilität bereits sehr zufriedenstellend. Sie besitzen jedoch ebenso, wie andere aus dem Stand der Technik bekannte heterogene Oxidationskatalysatoren den Nachteil, ein teures Edelmetall als katalytisch aktiven Bestandteil zu enthalten. Dies stellt insbesondere für die industrielle Großproduktion der Oxidationsprodukte, wie z.B. Propylenoxid, einen wesentlichen wirtschaftlichen Nachteil dar.

Es ist somit Aufgabe der vorliegenden Erfindung, einen edelmetallfreien heterogenen Katalysator bereitzustellen, der außerdem im wesentlichen frei von Halogenatomen ist und sowohl bei der Herstellung von Wasserstoffperoxid als auch bei der katalytischen Oxidation von organischen Molekülen, wie insbesondere bei der Epoxidation von Olefinen, anwendbar ist.

Überraschenderweise wird diese Aufgabe gelöst durch Bereitstellung einer festen Katalysatorzusammensetzung, die eine Nichtedelmetallkomponente, Phosphat sowie eine Stickstoffkomponente als wesentliche Bestandteile umfaßt.

Erfindungsgemäß geeignete Metalle sind die d- und f-Elemente d.h. Elemente der 4. bis 6. Periode aus den Gruppen IIIB, IVB, VB, VIB, VIIB, IB, IIB, IIIA, IVA und VA des Periodensystems, d.h. Sc, Ti, V, Cr, Mn, Cu, Zn, Ga, Ge, Y, Zr, Nb, Mo, Cd, In, Sn, Sb, La, Ce, Pr, Nd, Sm, Eu, Gd, Tb, Dy, Ho, Er, Tm, Yb, Lu Hf, Ta, W, Re, Tl, Pb, Bi und zusätzlich Fe, Co und Ni.

Gegenstand der Erfindung ist somit eine Katalysatorzusammensetzung (im folgenden als Metallphosphat bezeichnet), die dadurch erhältlich ist, daß man
a) ein wäßriges Gemisch herstellt, das
   i) ein Salz mindestens eines Nichtedelmetalls, ausge-wählt unter Elementen mit den Ordnungszahlen 21 bis 32, 39 bis 42, 48 bis 51, 57 bis 75 und 81 bis 83;
   ii) mindestens ein Phosphation und
   iii) mindestens eine Stickstoffquelle enthält; und
b) die so erhaltene wäßrige Lösung einengt und die dabei anfallende Katalysatormasse, gegebenenfalls unter leichtem Erwärmen, unter Beibehaltung ihrer katalytischen Aktivität trocknet;
wobei in dem katalytisch aktiven, getrockneten Feststoff Nichtedelmetall (M), Phosphat (P) und Stickstoff (N) in einem molaren Verhältnis von M:P:N = 1 : 0,9 bis 1,3: 0,9 bis 1,7 vorliegen.

Das wäßrige Gemisch der Stufe a) erhält man vorzugsweise dadurch, daß man die Komponenten i), ii) und iii) in einem wäßrigen Lösungsmittel, wie z.B. Wasser oder einem wäßrig-alkoholischen, wie etwa einem wäßrig-ethanolischen Lösungsmittel, löst. Wasser ist jedoch als Lösungsmittel bevorzugt. Die Komponenten können gemeinsam oder getrennt voneinander gelöst werden. Vorzugsweise stellt man jedoch getrennt voneinander zwei Lösungen her, wobei eine davon das Nichtedelmetallsalz und die andere die Posphatkomponente enthält, und vereinigt anschließend die beiden Lösungen. Die erforderliche Stickstoffquelle kann in einer oder beiden dieser Lösungen enthalten sein.

Bei der Herstellung der erfindungsgemäßen Metallphosphat-Katalysatoren ist es von Vorteil, die Metallkomponente in wäßriger Lösung in Form leicht löslicher Salze vorzulegen und das Phosphat in gelöster Form unter gleichmäßigem Rühren hinzuzufügen.

Die Wahl des geeignetsten pH- und Temperaturbereiches für die Herstellung der jeweiligen Katalysatorzusammensetzung liegt im Bereich fachmännischen Könnens. Bei der Herstellung der Teillösungen, bzw. des wäßrigen Gemischs ist es gewöhnlich ausreichend, bei Temperaturen im Bereich von 10 bis 60°C, vorzugsweise bei etwa 20 bis 30°C zu arbeiten. Je nach Lösungsverhalten der eingesetzten Komponenten kann aber auch ein Erwärmen einer Teillösung oder des wäßrigen Gemisches über den obigen Wert hinaus angebracht sein. Während der Herstellung der Teillösungen bzw. des Gemisches sind gewöhnlich keine besonderen Maßnahmen zur pH-Wert-Einstellung zu ergreifen. Je nach Lösungserhalten einzelner Komponenten kann jedoch die Zugabe pH-Wert-einstellender Mittel, wie z.B. üblicher Säuren oder Basen, oder üblicher Puffersubstanzen, von Vorteil sein.

Vorzugsweise enthält das gemäß Stufe a) erzeugte wäßrige Gemisch Nichtedelmetall-Ionen (M), wie z.B. Metallkationen, Phosphat (P) und Stickstoffquelle (N) in einem molaren Verhältnis im Bereich im Bereich von etwa 1 : 0,8 bis 1,4 : 0,8 bis 4,0, wie z.B. 1:1:1 oder 1:1:4.

Die jeweilige Konzentration der einzelnen im erfindungsgemäßen wäßrigen Gemisch enthaltenen Komponenten kann in einem weiten Bereich schwanken und wird im wesentlichen von der Löslichkeit der verwendeten Verbindungen bestimmt. Es ist jedoch von Vorteil möglichst konzentrierte wäßrige Lösungen herzustellen, um Zeit- und Energiebedarf für das Einengen des wäßrigen Gemischs möglichst gering zu halten, vorausgesetzt die Bildung des erfindungsgemäßen katalytisch aktiven Metallphosphats wird dadurch nicht beeinträchtigt. So können beispielsweise Metallkomponente und Phosphatkomponente unabhängig voneinander in einer Konzentration im Bereich von etwa 0,1 bis etwa 1,5 Mol/l, wie z.B etwa 0,25 bis etwa 0,85 Mol/l, vorliegen. Die Stickstoffquelle(n) kann (können) beispielsweise in einer Konzentration im Bereich von etwa 0,1 bis etwa 5 Mol/l, wie z.B. etwa 0,25 bis etwa 3,5 M, vorliegen. Bei Verwendung von Ammoniumionen als Stickstoffquelle liegen Metallkomponente, Phosphat und Ammonium vorzugsweise in etwa äquimolaren Mengen im Gemisch vor, wobei die Konzentration jeder der drei Komponenten bei etwa 0,25 bis etwa 0,85 Mol/l liegen kann.

Während der Einengung und Trocknung des wäßrigen Gemischs werden die Bedingungen vorzugsweise so gewählt, daß ein vollständiges Entweichen der Stickstoffkomponente aus der Katalysatormasse im wesentlichen vermieden wird. Insbesondere sollten die Bedingungen so gewählt sein, daß der Stickstoffanteil in der Katalysatormasse nach Beendigung der Trocknung um nicht mehr als etwa 20 bis 90 Mol-%, vorzugsweise etwa 50 - 80 Mol-%, bezogen auf den eingesetzten Stickstoff, verringert ist.

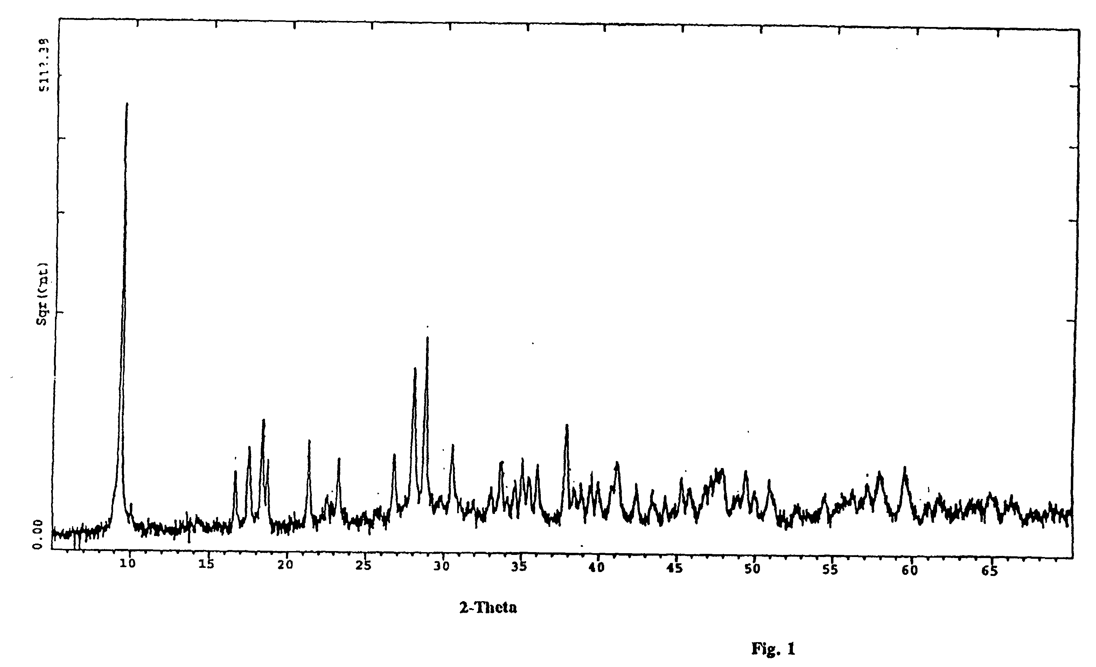

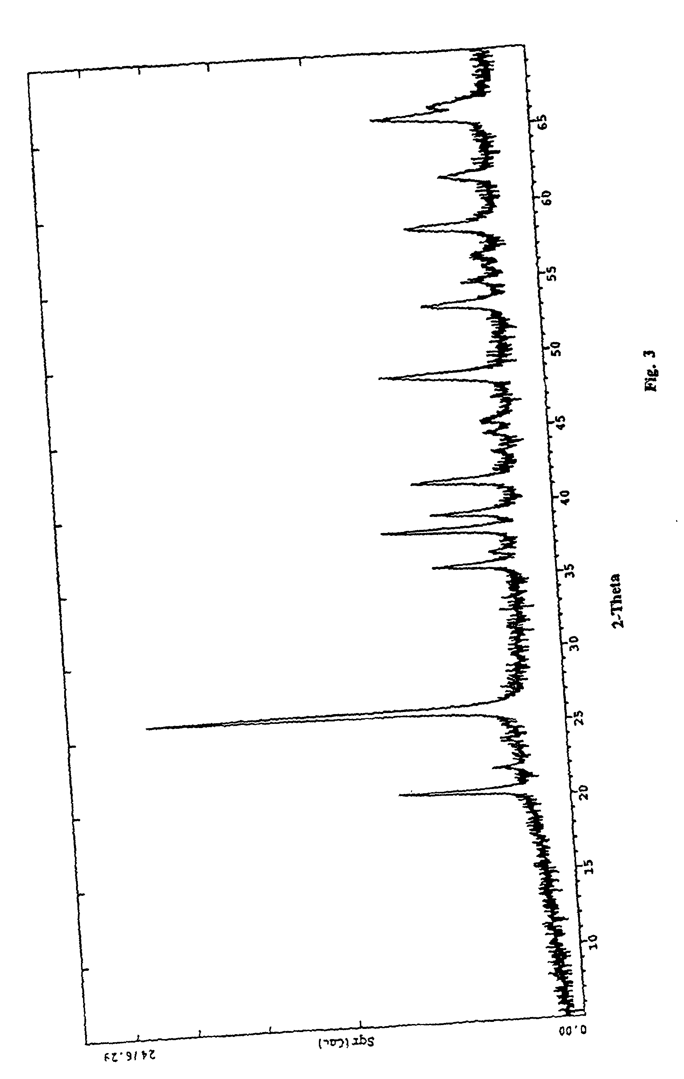

Die Wahl der für das jeweilige Katalysatormaterial geeignetsten Trocknungsbedingungen liegt im Bereich fachmännischen Könnens. Wie die beiliegenden Beispiele veranschaulichen, kann'beispielsweise ein Trocknen der Katalysatormasse bei zu hoher Temperatur einen vollständigen Stickstoffverlust bewirken. Dieser Verlust ist über eine deutliche, charakteristische Veränderung in den Röntgenbeugungsbildern der Feststoffmasse nachweisbar, wie ein Vergleich der beigefügten Röntgendiffraktogramme (Figur 1 und Figur 3) zeigt. Insbesondere ist die für die erfindungsgemäßen katalytisch aktiven Phosphate auffällige Feinstruktur im Diffraktogramm nicht mehr nachweisbar. Vor allem führt der Stickstoffverlust aber zu einer Abnahme bzw. vollständigen Zerstörung der katalytischen Aktivität.

Man erhält das erfindungsgemäße, katalytisch aktive Metallphosphat beispielsweise dann, wenn man das wäßrige Gemisch in einem Druckbereich von etwa 10 bis 1000 mbar, wie z.B. etwa 15 - 50 mbar, bei einer Temperatur im Bereich von etwa 10 bis etwa 200°C, wie z.B. etwa 100 - 140°C zunächst zur Trockene einengt und den auf diese Weise erhaltenen Rückstand bei einer Temperatur im Bereich von etwa 30 bis etwa 200°C, vorzugsweise etwa 50 bis etwa 150°C, insbesondere etwa 60 bis etwa 140°C, wie z.B. 120°C, unter Luft und bei Normaldruck trocknet. Die Trocknungsdauer kann dabei etwa 5 bis 20 Stunden, wie z.B. etwa 8 bis 12 Stunden, betragen.

Auf diese Weise entstehen Festkörperphasen, die in der Lage sind, ohne Edelmetalle und halogenhaltige Promotoren, Wasserstoffperoxid aus Wasserstoff und Sauerstoff durch heterogene Katalyse zu bilden.

In der so hergestellten Katalysatorzusammensetzung können Nichtedelmetall (M), Phosphat (P) und Stickstoff (N) in einem molaren Verhältnis von M:P:N = 1:0,9 bis 1,3:0,9 bis 1,7, wie z.B. im Verhältnis 1:1 bis 1,3:1,1 bis 1,5, oder etwa 1:1,1 bis 1,2:1,1 bis 1,5 vorliegen.

Besonders bevorzugt setzt man zur Herstellung des wäßrigen Gemischs gemäß Stufe a) wasserlösliche Nichtedelmetallsalze, wie z.B. Halogenide, wie Fluoride, Bromide oder Chloride, Hydroxide, Nitrate, Sulfate, Cyanide oder andere wasserlösliche Salze ein. Die Verwendung von Nitraten ist dabei besonders bevorzugt. Als Nichtedelmetall finden insbesondere Elemente mit den Ordnungszahlen 21 bis 32, 39 bis 42 und 48 bis 51 Verwendung. Die Oxidationsstufe des Metallions kann dabei variieren und z.B. +1, +2, +3, +4, +5, +6 oder +7 betragen. Bevorzugt sind jedoch solche Oxidationsstufen, von denen wasserlösliche Salze existieren.

Gemäß besonders bevorzugter Ausführungsformen verwendét man Salze von Eisen in den Oxidationsstufen +2, +3, +4, +5 oder +6, insbesondere +2 oder +3 sowie Salze des Zinns in den Oxidationsstufen +2 oder +4, insbesondere +2.

Am meisten bevorzugt ist die Verwendung von wasserlöslichen Eisensalzen, wie z.B. Eisen-(III)-nitrat, und wasserlöslichen Zinnsalzen, wie Zinn-(II)-chlorid.

Als Phosphatkomponente sind erfindungsgemäß anwendbar Meta- und Orthophosphorsäure und die wasserlöslichen, edelmetallfreien Salze davon. Besonders bevorzugt ist die Verwendung wasserlöslicher Salze der Orthophosphorsäure, die in wäßriger Lösung Phosphat-, Hydrogenphosphat- oder Dihydrogenphosphationen bilden.

Als Stickstoffquelle sind erfindungsgemäß anwendbar Salpetersäure und die wasserlöslichen, edelmetallfreien Salze davon. Als bevorzugte Beispiele können genannt werden wasserlösliche Nitratsalze der oben aufgeführten Nichtedelmetalle. Außerdem sind anwendbar Ammoniak und die wasserlöslichen, edelmetallfreien Salze davon. Weiterhin sind einsetzbar primäre, sekundäre oder tertiäre Amine oder die Salze davon, welche in dem erfindungsgemäß verwendeten Lösungsmittel löslich sind. Als Beispiele können genannt werden Niedrikalkylamine mit bis zu 3 und Niedrigalkylammoniumsalze mit bis zu 4 Niedrigalkylgruppen. Die Niedrigalkylgruppen stehen dabei vorzugsweise für C₁-C₄-Alkylgruppen, wie Methyl, Ethyl, n-Propyl und n-Butyl.

Vorzugsweise erfolgt die Herstellung der erfindungsgemäßen stickstoffhaltigen Metallphosphate unter Verwendung von Ammonium- oder Niedrigalkylammoniumphosphaten. Besonders bevorzugt verwendet man Ammoniumdihydrogenphosphat.

Gemäß einer speziellen Ausführungsform der vorliegenden Erfindung erhält man aus Eisen-(III)-nitrat und Ammoniumdihydrogenphosphat nach Trocknen eine Katalysatorzusammensetzung, die ein Röntgendiffraktogramm zeigt, das folgende charakteristischen Beugungslinien umfaßt:

| 2-Theta | d |
|---|---|
| 9,37 | 9,429 |
| 18,37 | 4,824 |
| 28,01 | 3,183 |
| 28,78 | 3,099 |
| 35,05 | 2,558 |
| 37,87 | 2,373 |

Gemäß einer weiteren bevorzugten Ausführungsform der Erfindung erhält man aus Zinn-(II)-chlorid und Ammoniumdihydrogenphosphat nach dem Trocknen eine Katalysatorzusammensetzung, die ein Röntgendiffraktogramm zeigt, das folgende charakteristischen Beugungslinien umfaßt:

| 2-Theta | d |
|---|---|
| 12,79 | 6,915 |
| 13,04 | 6,784 |
| 19,09 | 4,645 |
| 20,21 | 4,389 |
| 23,01 | 3,861 |
| 23,90 | 3,720 |
| 26,18 | 3,400 |
| 30,33 | 2,944 |

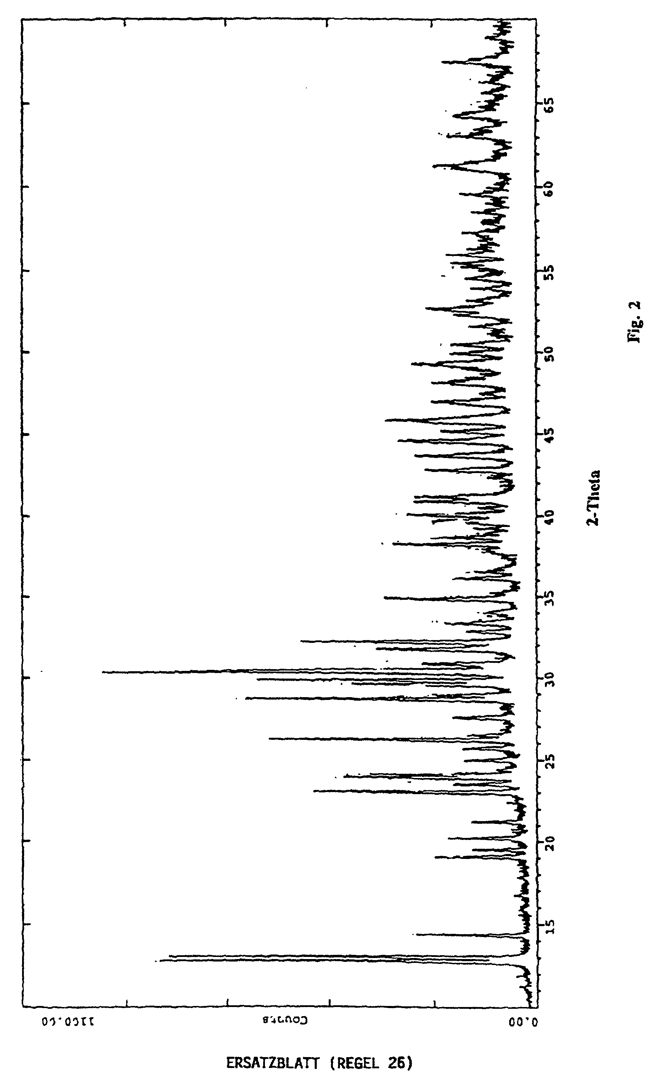

Die oben angegebenen 2-Theta-Werte wurden unter Verwendung der-Kupfer-K(α)-Strahlung (Wellenlänge 1: 1,54056 Angström; Wellenlänge 2: 1,54439 Angström) bestimmt. Weitere Beugungslinien können den beiliegenden Figuren 1 und 2 entnommen werden.

Gemäß einer weiteren bevorzugten Ausführungsform wird das erfindungsgemäße katalytisch aktive Metallphosphat mit einem Sauerstoffüberträger als weiterer katalytisch aktiver Komponente kombiniert. Hierzu kann man beispielsweise den in der Regel festen Sauerstoffüberträger in der gemäß obiger Stufe a) hergestellten wäßrigen Metallsalzlösung suspendieren und die auf diese Weise erhaltene Suspension, wie oben beschrieben, einengen und trocknen.

Während das erfindungsgemäße Metallphosphat insbesondere zur Verwendung in Verfahren zur Herstellung von Wasserstoffperoxid geeignet ist, findet das mit dem Sauerstoffüberträger kombinierte Metallphosphat vorzugsweise Verwendung als heterogener Katalysator in organischen Oxidationsreaktionen, wie beispielsweise bei der Epoxidation von Olefinen.

Gegenstand der Erfindung ist somit auch ein Verfahren zur Herstellung von Wasserstoffperoxid, wobei man unter konventionellen Bedingungen Wasserstoff und Sauerstoff in Gegenwart vón erfindungsgemäßem Metallphosphat umsetzt und das gebildete Wasserstoffperoxid von der Katalysatormasse abtrennt.

Gegenstand der Erfindung ist außerdem ein Verfahren zur Epoxidation von Olefinen, das dadurch gekennzeichnet ist, daß man das Olefin in Gegenwart von Wasserstoff und Sauerstoff katalytisch umsetzt. Das eingesetzte Olefin kann eine beliebige organische Verbindung sein, die mindestens eine ethylenisch ungesättigte Doppelbindung enthält. Sie kann aliphatischer, aromatischer oder cycloaliphatischer Natur sein, sie kann aus-einer linearen oder einer verzweigten Struktur bestehen. Vorzugsweise enthält das Olefin 2 bis 30 C-Atome. Mehr als eine ethylenisch ungesättigte Doppelbindung kann vorhanden sein, so wie etwa in Dienen oder Trienen. Das Olefin kann zusätzlich funktionelle Gruppen, wie Halogenatome, Carboxylgruppen, Carbonesterfunktionen, Hydroxylgruppen, Etherbrücken, Sulfidbrücken, Carbonylfunktionen, Cyanogruppen, Nitrogruppen oder Aminogruppen enthalten.

Typische Beispiele für derartige Olefine sind Ethylen, Propen, 1-Buten, cis- und trans-2-Buten, 1,3-Butadien, Pentene, Isopren, Hexene, Octene, Nonene, Decene, Undecene, Dodecene, Cyclopenten, Cyclohexen, Dicyclopentadien, Methylencyclopropan, Vinylcyclohexan, Vinylcyclohexen, Allylchlorid, Acrylsäure, Methacrylsäure, Crotonsäure, Vinylessigsäure, Allylalkohol, Alkylacrylate, Alkylmethacrylate, Ölsäure, Linolsäure, Linolensäure, Ester und Glyceride derartiger ungesättigter Fettsäuren, Styrol, α-Methylstyrol, Divinylbenzol, Inden und Stilben. Auch Mischungen der genannten Olefine können nach dem erfindungsgemäßen Verfahren epoxidiert werden.

Das erfindungsgemäße Verfahren eignet sich in besonderem Maße für die Epoxidierung von Propen zu Propylenoxid. Als Katalysator verwendet man hierzu vorteilhafterweise das mit dem Sauerstoffüberträger kombinierte erfindungsgemäße Metallphosphat. Während die Metallphosphatkomponente die in situ-Produktion von Wasserstoffperoxid katalysiert, wird mit Hilfe der Überträgerkomponente das Olefin epoxidiert.

Dabei ist es wirtschaftlich vorteilhaft, die Reaktion nur in einem Druckbereich von etwa 1 - 20 bar bei Temperaturen von etwa 5 - 70°C, insbesondere bei etwa 20 - 55°C ablaufen zu lassen. Das Molverhältnis von H₂:O₂ kann im Bereich von etwa 1:1 bis etwa 1:20, insbesondere von etwa 1:1 bis etwa 1:10, variiert werden.

Als Sauerstoffüberträger sind in den erfindungsgemäßen Katalysatoren beispielsweise Titansilikate mit Pentasilstruktur anwendbar. Insbesondere sind als Beispiele für Silikate solche mit röntgenografischer Zuordnung zur MFI-, MEL- oder MFI/MEL-Mischstruktur zu nennen. Zeolithe dieses Typs sind beispielsweise beschrieben in W.M. Meier, D.H. Olson, "Atlas of Zeolite Structure Types", Butterworths, 2. Aufl., 1987. Brauchbar sind weiterhin titanhaltige Zeolithe mit der Struktur des ZSM-48, Ferrierit, ZSM-12 oder β-Zeolith. Anstelle des Titans kann z.B. auch Vanadium im Zeolithen gebunden vorliegen. Ebenso können titan-, vanadium-, molybdän-, rhenium- oder wolframhaltige mesoporöse Oxide nach US 5057296 oder DE-OS 4407326 eingesetzt werden.

Die obengenannten, besonders bevorzugten Titansilikate mit MEI-Pentasilstruktur werden hergestellt, indem man ein Synthesegel bestehend aus Wasser, einer Titanquelle und Siliziumdioxid in geeigneter Weise unter Zusatz von organischen, stickstoffhaltigen Verbindungen unter hydrothermalen Bedingungen und gegebenenfalls unter Zusatz von Ammoniaklösung, Alkali oder Fluorid als Mineralisator kristallisiert. Als stickstoffhaltige Verbindung kommen z.B. 1,6-Diaminohexan (vgl. EP 0 007 081) oder vorzugsweise die Salze, bzw. das freie Hydroxid der Tetraalkylammoniumsalze, wie insbesondere des Tetrapropylammoniums (TPA) (vgl. DE-OS 3047798), in Frage. Wie in der DE-OS 4138155 beschrieben, kann auf den Einsatz von teurem TPAOH verzichtet werden, wenn dafür TPABr zusammen mit Ammoniak eingesetzt wird. Insbesondere letztgenannte Methode vermeidet eine Alkalikontamination des Titansilikats; Alkaligehalte <100 ppm sind erstrebenswert, um später einen hinreichend aktiven Epoxidationskatalysator zu erhalten.

Die Kristallisation der phasenreinen Struktur des Titansilikats mit MFI-Struktur erfolgt vorzugsweise bei Temperaturen von 140-190°C, insbesondere vorteilhaft bei 175°C innerhalb von etwa 2 bis 7 Tagen, wobei bereits nach ca. 4 Tagen gut kristallines Produkt erhalten wird. Durch starkes Rühren und hohen pH-Wert von etwa 12-14 während der Kristallisation kann die Synthesedauer einerseits und die Kristallitgröße andererseits deutlich verringert werden.

Von Vorteil sind beispielsweise Primärkristallite mit einer Partikelgröße von 0.05 bis 0.5 µm, insbesondere aber weniger als 0.2 µm.

Nach der Kristallisation kann das Titansilikat nach an sich bekannten Methoden abfiltriert, gewaschen und bei 100-120°C getrocknet werden. Zur Entfernung der in den Poren noch vorliegenden Amin- oder Tetraalkylaznmoniumverbindung kann das Material noch einer thermischen Behandlung an Luft oder unter Stickstoff unterzogen werden. Dabei ist es vorteilhaft, den Temperaturanstieg auf Werte <550°C begrenzen.

Das Vorliegen der für die Olefinoxidation benötigten Katalysatorfunktionen kann auch durch IR-Spektroskopie geprüft werden; bei etwa 550 cm⁻¹ and bei etwa 960 cm⁻¹ treten signifikante Banden auf, die das Vorliegen der erwünschten Festkörper-Kristallinität, sowie der benötigten Epoxidationsaktivität anzeigen.

Die auf diese Weise hergestellten Titanzeolithe können gemäß einer bevorzugten Ausführungsform den erfindungsgemäßen Metallphosphaten zugesetzt werden. Beispielsweise kann man hierzu die Lösung eines Metallnitrates und Ammoniumphosphates vorlegen und dann portionsweise den frisch kalzinierten Titanzeolithen unter Rühren zusetzen. Die Zeolith-Suspension kann man dann bei Temperaturen von etwa 30 - 200°C, insbesondere etwa 50 bis 100°C und gegebenenfalls unter reduziertem Druck einengen.

Zur Modifizierung der erfindungsgemäßen Katalysator-Zusammensetzungen können die aus dem Stand der Technik bekannten Methoden angewendet werden. Als Beispiele sind zu nennen die Verformung unter Zuhilfenahme eines Bindemittels, der Ionenaustausch und/oder die Imprägnierung mit Metallen, die Oberflächenmodifizierung, wie beispielsweise über CVD (Chemical Vapor Deposition) oder die chemische Derivatisierung, wie etwa eine Silylierung. Außerdem ist denkbar, die erfindungsgemäße Katalysatorzusammensetzung auf einem festen, inerten Träger abzuscheiden. Als inerte Träger sind beispielsweise geeignet Kügelchen, Pellets oder Stränge aus Aluminiumoxid oder Siliciumdioxid. Zur Herstellung der erfindungsgemäßen, trägergebundenen Katalysatorzusammensetzung kann man beispielsweise die Trägerpartikel der obenbeschriebenen wässrigen Metallsalzlösung vor dem Einengen, gegebenenfalls zusammen mit dem obenbeschriebenen Sauerstoffüberträger, zusetzen und das Gemisch, wie oben beschrieben, einengen und trocknen.

In Abhängigkeit vom umzusetzenden organischen Molekül können die erfindungsgemäßen Katalysatoren in Flüssig- oder Gasphase oder aber auch in überkritischer Phase eingesetzt werden. Dabei wird der Katalysator bei Flüssigphasen vorzugsweise als Suspension eingesetzt, während bei Gasphasen- oder überkritischer Fahrweise eine Festbettanordnung von Vorteil ist.

Desaktivierte Katalysatoren können durch kontrolliertes Abbrenner von Kohlenstoffbelegungen und nachfolgende Reduktion, wie z.B. mit Wasserstoff, wieder in eine aktive Form zurückgeführt werden. Bei geringer Belegung kann der Katalysator auch durch einen einfachen Waschprozeß wieder regeneriert werden. Je nach Bedarf kann der Waschvorgang im neutralen, sauren oder basischen pH-Bereich durchgeführt werden. Gegebenenfalls kann auch mittels'einer mineralsauren Wasserstoffperoxidlösung die Katalysatoraktivität wieder regeneriert werden.

Die vorliegende Erfindung wird nun anhand der folgenden Beispiele weiter erläutert.

### Beispiel 1

### Herstellung eines Eisenphosphat-Katalysators (Katalysator A)

In einem Polypropylen-Becher werden bei Raumtemperatur 116 g (0,33 Mol) Eisen-III-nitrat-Hexahydrat (Riedel de Haen) in 250 ml deionisiertem Wasser gelöst und in einen 1 1-Glasrührkolben überführt. Getrennt davon löst man bei Raumtemperatur 38,3 g (0,33 Mol) Ammoniumdihydrogenphosphat (NH₄H₂PO₄) (Merck) in 150 ml deionisiertem Wasser und tropft unter kräftigem Rühren die gebildete Phosphatlösung zur Eisennitrat-Lösung.

Die so entstandene Lösung wird noch eine Stunde bei Raumtemperatur gerührt. Die rötliche Lösung wird dann in einen Rotavap überführt und bei 90°C und 15-20 mbar einrotiert. Der erhaltene Feststoff wird über Nacht bei 120°C in einem Umluft-Trockenschrank unter Luft getrocknet. Das Produkt zeigt das in Figur 1 angegebene Röntgendiffraktogramm. Die ermittelten 2-Theta-Werte sowie die dazugehörigen d-Werte und die relativen Intensitäten für die ermittelten Beugungslinien sind in folgender Tabelle I zusammengefaßt.

**Tabelle I**

| Peak-Nummer | 2-Theta^{a} | d | % | Peak-Nummer | 2-Theta^{a} | d | % |
|---|---|---|---|---|---|---|---|
| 1 | 9.372 | 9.4287 | 100.00 | 28 | 38.864 | 2.3153 | 2.46 |
| 2 | 10.063 | 8.7830 | 1.08 | 29 | 39.445 | 2.2825 | 2.27 |
| 3 | 14.270 | 6.2015 | 0.58 | 30 | 39.540 | 2.2773 | 2.24 |
| 4 | 16.698 | 5.3049 | 3.13 | 31 | 39.979 | 2.2533 | 2.54 |
| 5 | 17.546 | 5.0504 | 5.45 | 32 | 40.858 | 2.2068 | 1.99 |
| 6 | 18.378 | 4.8236 | 8.66 | 33 | 41.172 | 2.1907 | 4.01 |
| 7 | 18.723 | 4.7354 | 4.23 | 34 | 42.423 | 2.1290 | 2.43 |
| 8 | 21.371 | 4.1543 | 5.98 | 35 | 43.475 | 2.0799 | 1.19 |
| 9 | 22.564 | 3.9373 | 1.60 | 36 | 44.307 | 2.0427 | 1.36 |
| 10 | 22.878 | 3.8839 | 1.16 | 37 | 45.296 | 2.0004 | 2.85 |
| 11 | 23.318 | 3.8117 | 4.15 | 38 | 45.845 | 1.9777 | 1.91 |
| 12 | 25.657 | 3.4692 | 0.64 | 39 | 46.866 | 1.9370 | 2.05 |
| 13 | 26.803 | 3.3235 | 4.81 | 40 | 47.211 | 1.9236 | 2.99 |
| 14 | 28.012 | 3.1827 | 17.15 | 41 | 47.572 | 1.9098 | 3.49 |
| 15 | 28.781 | 3.0994 | 23.32 | 42 | 47.980 | 1.8945 | 3.60 |
| 16 | 29.827 | 2.9930 | 1.52 | 43 | 48.885 | 1.8616 | 1.38 |
| 17 | 30.518 | 2.9268 | 5.15 | 44 | 49.403 | 1.8432 | 3.37 |
| 18 | 31.884 | 2.8045 | 1.36 | 45 | 49.984 | 1.8232 | 1.72 |
| 19 | 33.061 | 2.7072 | 1.72 | 46 | 50.926 | 1.7916 | 2.77 |
| 20 | 33.673 | 2.6594 | 4.07 | 47 | 52.684 | 1.7359 | 0.97 |
| 21 | 34.097 | 2.6273 | 1.55 | 48 | 54.474 | 1.6830 | 1.44 |
| 22 | 34.553 | 2.5937 | 2.63 | 49 | 55.583 | 1.6520 | 1.08 |
| 23 | 35.055 | 2.5577 | 3.87 | 50 | 56.274 | 1.6334 | 2.13 |
| 24 | 35.505 | 2.5263 | 2.77 | 51 | 57.138 | 1.6107 | 2.13 |
| 25 | 36.054 | 2.4890 | 3.85 | 52 | 57.938 | 1.5904 | 3.02 |
| 26 | 37.875 | 2.3734 | 8.33 | 53 | 59.477 | 1.5529 | 3.54 |
| 27 | 38.409 | 2.3417 | 1.88 | 54 | 60.963 | 1.5185 | 1.41 |
| | | | | 55 | 61.654 | 1.5031 | 1.38 |
| | | | | 56 | 64.950 | 1.4346 | 1.58 |
| | | | | 57 | 66.206 | 1.4104 | 1.88 |

| | | | | | | | |
|---|---|---|---|---|---|---|---|
| a Die oben angegebenen 2-Theta-Werte wurden unter Verwendung der Kupfer-K(α)-Strahlung (Wellenlänge 1: 1,54056 Angström;Wellenlänge 2: 1,54439 Angström) bestimmt. | | | | | | | |

Der Katalysator enthält 22,2 Gew.-% Eisen, 14,0 Gew.-% Phosphor und 8,3 Gew.-% Stickstoff, was einem molaren Verhältnis von Fe:P:N von etwa 1:1,13:1,5 entspricht.

### Beispiel 2

### Verwendung des erfindungsgemäßen Katalysators A zur katalytischen Erzeugung von Wasserstoffperoxid aus den Elementen.

In einem Stahlautoklaven mit Glaseinsatz (25 ml Fassungsvermögen) wird der Katalysator aus Beispiel 1 (100 mg) in 10 ml Methanol vorgelegt und der Autoklav verschlossen. In einer explosionsgeschützten Vorrichtung wird bei 27°C unter Rühren Wasserstoff zugefahren (30 min; 10 ml/min). Dann wird der Druck mit Stickstoff auf 40 bar erhöht und abschließend Sauerstoff (100 ml/min) hinzu dosiert. Nach einer Reaktionsdauer von 4 Stunden wird der Autoklav langsam entspannt und der Inhalt analysiert. Mittels jodometrischer Titration werden 0,70 Gew.-% Wasserstoffperoxid bestimmt. Der Wassergehalt des Reaktionsaustrages beträgt 3,2 Gew.-%.

### Beispiel 3

### Herstellung eines Zinnphosphat-Katalysators (Katalysator B)

In einem Polypropylen-Becher werden bei Raumtemperatur 54,5 g (0,29 Mol) Zinn-(II)-chlorid (Merck) in 250 ml deionisiertem Wasser gelöst und in einen 2 1-Glasrührkolben überführt. Außerdem löst man bei Raumtemperatur 38,3 g (0,33 Mol) Ammoniumdihydrogenphosphat (Merck) in 950 ml deionisiertem Wasser und tropft die Phosphatlösung unter kräftigem Rühren zur Zinnchlorid-Lösung. Die entstandene Suspension wird noch für die Dauer von einer Stunde bei Raumtemperatur gerührt. Danach wird der Ansatz in einen Rotavap überführt und bei 90°C und 20 mbar eingedampft und anschließend mit H₂O chloridfrei gewaschen. Der erhaltene Feststoff wird über Nacht bei 120°C in einem Umluft-Trockenschrank unter Luft getrocknet. Das Produkt zeigt das in Figur 2 angegebene Röntgendiffraktogramm. Die ermittelten 2-Theta-Werte sowie die dazugehörigen d-Werte und die relativen Intensitäten für die ermittelten Beugungslinien sind in folgender Tabelle II zusammengefaßt.

**Tabelle II**

| Peak-Nummer | 2-Theta^{a} | d | % | Peak-Nummer | 2-Theta^{a} | d | % |
|---|---|---|---|---|---|---|---|
| 1 | 11.207 | 7.8890 | 3.77 | 24 | 28.986 | 3.0779 | 20.67 |
| 2 | 11.905 | 7.4280 | 4.84 | 25 | 29.578 | 3.0177 | 42.09 |
| 3 | 12.792 | 6.9147 | 85.68 | 26 | 29.838 | 2.9919 | 63.83 |
| 4 | 13.040 | 6.7835 | 84.93 | 27 | 30.335 | 2.9441 | 100.00 |
| 5 | 14.365 | 6.1607 | 26.59 | 28 | 30.867 | 2.8945 | 26.37 |
| 6 | 16.719 | 5.2982 | 5.27 | 29 | 31.742 | 2.8166 | 36.38 |
| 7 | 17.827 | 4.9713 | 4.41 | 30 | 32.180 | 2.7793 | 53.61 |
| 8 | 19.093 | 4.6445 | 23.14 | 31 | 32.850 | 2.7241 | 16.04 |
| 9 | 19.507 | 4.5469 | 14.75 | 32 | 33.347 | 2.6847 | 17.55 |
| 10 | 20.217 | 4.3888 | 20.34 | 33 | 33.797 | 2.6500 | 7.75 |
| 11 | 21.210 | 4.1854 | 13.89 | 34 | 34.033 | 2.6321 | 10.66 |
| 12 | 23.016 | 3.8609 | 50.81 | 35 | 34.861 | 2.5714 | 34.55 |
| 13 | 23.501 | 3.7823 | 19.16 | 36 | 35.228 | 2.5455 | 10.33 |
| 14 | 23.903 | 3.7196 | 44.03 | 37 | 36.115 | 2.4850 | 19.16 |
| 15 | 24.093 | 3.6908 | 38.00 | 38 | 36.541 | 2.4570 | 13.02 |
| 16 | 24.365 | 3.6502 | 8.72 | 39 | 36.754 | 2.4432 | 8.72 |
| 17 | 24.980 | 3.5617 | 16.68 | 40 | 37.464 | 2.3986 | 7.75 |
| 18 | 25.678 | 3.4664 | 16.58 | 41 | 37.744 | 2.3814 | 13.35 |
| 19 | 26.187 | 3.4002 | 57.59 | 42 | 37.898 | 2.3721 | 10.44 |
| 20 | 26.482 | 3.3629 | 15.93 | 43 | 38.241 | 2.3516 | 32.72 |
| 21 | 27.531 | 3.2372 | 18.95 | 44 | 38.678 | 2.3260 | 26.70 |
| 22 | 28.655 | 3.1127 | 66.52 | 45 | 38.927 | 2.3117 | 10.55 |
| 23 | 28.915 | 3.0853 | 22.82 | 46 | 39.223 | 2.2950 | 15.61 |

| | | | | | | | |
|---|---|---|---|---|---|---|---|
| a Die oben angegebenen 2-Theta-Werte wurden unter Verwendung der Kupfer-K(α)-Strahlung (Wellenlänge 1: 1,54056 Angström; Wellenlänge 2: 1,54439 Angström) bestimmt. | | | | | | | |

Der Katalysator enthält 37,0 Gew.-% Zinn, 11,2 Gew.-% Phosphor und 5,1 Gew.-% Stickstoff, was einem molaren Verhältnis von Sn:P:N von etwa 1:1,16:1,16 entspricht.

### Beispiel 4

### Verwendung des erfindungsgemäßen Katalysators B zur katalytischen Erzeugung von Wasserstoffperoxid aus den Elementen.

In einem Stahlautoklaven mit Glaseinsatz (25 ml Fassungsvermögen) wird der Katalysator aus Beispiel 3 (100 mg) in 10 ml Methanol vorgelegt und der Autoklav verschlossen. In einer explosionsgeschützten Vorrichtung wird bei 27°C unter Rühren Wasserstoff zugefahren (30 Minuten; 10 ml/min). Dann wird der Druck mit Stickstoff auf 40 bar erhöht und abschließend Sauerstoff (100 ml/min) hinzu dosiert. Nach einer Reaktionsdauer von 4 Stunden wird der Autoklav langsam entspannt und der Inhalt analysiert. Mittels jodometrischer Titration werden 0,38 Gew.-% Wasserstoffperoxid bestimmt. Der Wassergehalt des Reaktionsaustrages beträgt 1,1 Gew.-%.

### Beispiel 5

### Hersteliung eines erfindungsgemäß verwendbaren Titanzeolithen.

In einem Vierhalskolben (2 1 Inhalt) werden 455 g Tetraethylorthosilikat (Fa. Merck) bei Raumtemperatur vorgelegt und aus einem Tropftrichter innerhalb von 30 Minuten mit 15 g Tetraisopropylorthotitanat unter Rühren (250 U/min; Blattrüher) versetzt. Es bildet sich eine farblose, klare Mischung. Abschließend versetzt man mit 800 g einer Tetrapropylammoniumhydroxid-Lösung (40 % TPAOH, Fa. Alfa, verdünnt auf 20 Gew.-% mit deionisiertem Wasser, Alkaligehalt <10 ppm) und rührt noch eine Stunde nach. Bei 90° bis 100°C wird anschließend das durch Hydrolyse gebildete Alkoholgemisch (ca. 460 g) abdestilliert. Man füllt mit 1,5 1 entionisiertem Wasser auf und gibt das mittlerweile leicht opake Sol in einen 2,5 1 fassenden Rührautoklaven. Mit einer Heizrate von 3°C/min wird der verschlossene Autoklav (Ankerrührer, 200 U/min) auf eine Reaktionstemperatur von 175°C gebracht. Nach 92 Stunden wird die Reaktion durch Abkühlen beendet. Das erkaltete Reaktionsgemisch (weiße Suspension) wird abzentrifugiert und mehrfach mit Wasser bis zur Neutralität gewaschen. Der erhaltene Feststoff wird bei 110°C innerhalb von 24 Stunden getrocknet (Ausbeute 149 g). Abschließend wird unter Luft durch 5-stündiges Erhitzen auf 500°C das im Zeolithen noch vorhandene Templat abgebrannt (Kalzinierungsverlust: 14 Gew.-%).

Das reinweiße Produkt hat nach naßchemischer Analyse einen Ti-Gehalt von 1,5 Gew.-% und einen Restgehalt an Alkali (Kalium) unterhalb von <0,01 Gew.-%. Die Ausbeute beträgt, bezogen auf eingesetztes SiO₂, 97 %.

Die Kristallitgröße beträgt ca. 0,1-0,15 µm und das Produkt zeigt im IR-Spektrum typische Banden bei 960 cm⁻¹ und 550 cm⁻¹.

### Beispiel 6

### Herstellung eines erfindungsgemäßen Eisenphosphat- Epoxidationskatalysators

In einem Polypropylen-Becher löst man gemäß Beispiel 1 116 g (0,33 Mol) Eisen-(III)-nitrat (Riedel de Haen) in 250 ml deionisiertem Wasser. Getrennt davon löst man 38,3 g (0,33 Mol) Ammoniumdihydrogenphosphat in Wasser und gibt die Phosphatlösung unter Rühren zur vorgelegten Eisennitratlösung.

Die entstandene rosafarbene Lösung wird in einen Rotavap überführt. Zusätzlich gibt man eine Suspension von 7 g Titansilikalit aus Beispiel 5 in 50 ml deionisiertem Wasser hinzu und dampft die Suspension innerhalb von 5 Stunden wie in Beispiel 1 beschrieben ein. Abschließend wird der Katalysator über Nacht bei 120°C getrocknet.

Der Katalysator enthält 10,1 Gew.-% Eisen, 6,8 gew.-% Phosphor, 3,7 Gew.-% Stickstoff und 1,1 Gew.-% Titan.

### Beispiel 7

### Herstellung von Propylenoxid

In einer explosionsgeschützten Vorrichtung füllt man einen Glasdruckautoklaven mit 60 ml einer 50%-igen wäßrigen Methanollösung. Dazu gibt man 1 g des Katalysators aus Beispiel 6. Nach Erwärmen der katalysatorhaltigen Suspension im verschlossenen Autoklaven auf etwa 40 - 50°C dosiert man Stickstoff (30 ml/min), Sauerstoff (30 ml/min), Wasserstoff (60 ml/min), Propen (20 ml/min) bei einem konstant gehaltenen Druck von 1 bar zu. Im Abgasstrom des Reaktors mißt man nach 2 Stunden eine gaschromatographisch bestimmte C3-Fraktion von 101 ppm Propylenoxid neben 17,7 Vol.-% Propen und 0,11 Vol-% Propan. Diese Werte werden auch noch nach 6 Stunden beobachtet.

Nach Reaktionsende werden im flüssigen Reaktionsaustrag noch 260 ppm Propandiol nachgewiesen.

### Vergleichsbeispiel 1

### Einfluß der Trocknungstemperatur auf die katalytische Aktivität der erfindungsgemäßen Katalysatoren.

Man wiederholt Beispiel 1, mit Ausnahme davon, daß man den erhaltenen Feststoff zusätzlich bei 550°C unter Luft für die Dauer von 5 Stunden kalziniert.

Der Kalzinierungsverlust beträgt 58 Gew.-% bezogen auf die Einwaage. Stickstoff wird nicht mehr nachgewiesen. Das Produkt zeigt nunmehr das deutlich veränderte Röntgendiffraktorgramm gemäß Figur 3. Die ermittelten 2-Theta-Werte sowie die dazugehörigen d-Werte und die relativen Intensitäten für die ermittelten Beugungslinien sind in folgender Tabelle III zusammengefaßt.

**Tabelle III**

| Peak-Nummer | 2-Theta^{a} | d | % |
|---|---|---|---|
| 1 | 20.400 | 4.3499 | 17.26 |
| 2 | 21.922 | 4.0511 | 2.25 |
| 3 | 23.783 | 3.7382 | 2.19 |
| 4 | 25.897 | 3.4375 | 100.00 |
| 5 | 31.470 | 2.8404 | 1.42 |
| 6 | 35.615 | 2.5188 | 9.04 |
| 7 | 36.517 | 2.4586 | 2.25 |
| 8 | 38.096 | 2.3602 | 17.43 |
| 9 | 39.195 | 2.2965 | 8.87 |
| 10 | 41.394 | 2.1794 | 10.82 |
| 11 | 43.246 | 2.0903 | 1.54 |
| 12 | 44.543 | 2.0324 | 2.54 |
| 13 | 45.388 | 1.9965 | 2.19 |
| 14 | 48.546 | 1.8738 | 14.24 |
| 15 | 53.113 | 1.7229 | 8.33 |
| 16 | 54.824 | 1.6731 | 1.89 |
| 17 | 56.403 | 1.6300 | 1.65 |
| 18 | 58.292 | 1.5816 | 9.52 |
| 19 | 61.647 | 1.5033 | 4.73 |
| 20 | 65.650 | 1.4210 | 14.89 |
| 21 | 66.327 | 1.4081 | 5.44 |

| | | | |
|---|---|---|---|
| a Die oben angegebenen 2-Theta-Werte wurden unter Verwendung der Kupfer-K(α)-Strahlung (Wellenlänge 1: 1,54056 Angström;Wellenlänge 2: 1,54439 Angström) bestimmt. | | | |

### Vergleichsbeispiel 2

### Verwendung des Stickstoff-freien Vergleichskatalysators zur katalytischen Erzeugung von Wasserstoffperoxid aus den Elementen.

Man wiederholt Beispiel 2, wobei man jedoch nunmehr den Katalysator aus Vergleichsbeispiel 1 (100 mg) vorlegt. Nach einer Reaktionsdauer von 4 Stunden wird der Autoklav langsam entspannt und der Inhalt analysiert. Mittels jodometrischer Titration werden nur noch 0,17 Gew.-% Wasserstoffperoxid bestimmt. Der Wassergehalt des Reaktionsaustrages beträgt 2,1 Gew.-%.

### Vergleichsbeispiel 3

### Herstellung eines Phosphatkatalysators ohne erfindungsgemäße Metallkomponente.

In einem Polypropylen-Becher werden bei Raumtemperatur 18,9 g (0,3 Mol) Borsäure (Merck) in 250 ml deionisiertem Wasser gelöst und in einen 2 1-Glasrührkolben überführt. Getrennt davon löst man bei Raumtemperatur 38,3 g (0,33 Mol) Ammoniumdihydrogenphosphat (Merck) in 950 ml deionisiertem Wasser und tropft unter kräftigem Rühren die Phosphatlösung zur Borsäurelösung. Die entstandene Suspension wird noch für die Dauer von einer Stunde bei Raumtemperatur nachgerührt. Danach überführt man den Ansatz in einen Rotavap und dampft bei 90°C/20 mbar ein. Der erhaltene Feststoff wird über Nacht bei 120°C in einem Umluft-Trockenschrank unter Luft getrocknet.

Der Katalysator enthält 6,1 Gew.-% Bor, 20,7 Gew.% Phosphor und 9,6 Gew.-% Stickstoff.

### Vergleichsbeispiel 4

### Verwendung des Katalysators aus Vergleichsbeispiel 3 zur katalytischen Erzeugung von Wasserstoffperoxid aus den Elementen.

Man wiederholt Beispiel 2, wobei man jedoch nunmehr den Katalysator aus Vergleichsbeispiel 3 (100 mg) vorlegt. Nach einer Reaktionsdauer von 4 Stunden wird der Autoklav langsam entspannt und der Inhalt analysiert. Mittels jodometrischer Titration werden nur noch <0,01 Gew.-% Wasserstoffperoxid bestimmt. Der Wassergehalt des Reaktionsaustrages beträgt 0,6 Gew.-%.

## Patentansprüche

1. Edelmetallfreie Katalysatorzusammensetzung, dadurch erhältlich, daß man
a) ein wäßriges Gemisch herstellt, das
i) ein Salz mindestens eines Nichtedelmetalls, ausgewählt unter Elementen mit den Ordnungszahlen 21 bis 32, 39 bis 42, 48 bis 51, 57 bis 75 und 81 bis 83;
ii) Phosphationen; und
iii)mindestens eine Stickstoffquelle umfaßt; und
b) das so erhaltene wäßrige Gemisch einengt und die dabei anfallende Katalysatormasse trocknet,
wobei in dem katalytisch aktiven, getrockneten Feststoff Nichtedelmetall (M), Phosphat (P) und Stickstoff (N) in einem molaren Verhältnis von M:P:N = 1 : 0,9 bis 1,3: 0,9 bis 1,7 vorliegen.

2. Katalysatorzusammensetzung nach Anspruch 1, **dadurch gekennzeichnet, daß** die wäßrige Lösung Metallionen (M), Phosphationen (P) und Stickstoffquelle (N) in einem molaren Verhältnis von M : P : N = 1 : 0,8 bis 1,4 : 0,6 bis 4,0 enthält.

3. Ratalysatorzusammensetzung nach einem der vorhergehenden Ansprüche, **dadurch gekennzeichnet, daß** das Nichtedelmetallsalz ausgewählt ist unter wasserlöslichen Salzen von Metallen der Ordnungszahlen 21-32, 39-42 und 48-51.

4. Katalysatorzusammensetzung nach einem der vorhergehenden Ansprüche, **dadurch gekennzeichnet, daß** die Stickstoffquelle ausgewählt ist unter Salpetersäure und den edelmetallfreien, wasserlöslichen Salzen davon, Ammoniak, Aminen, Ammoniumoder C₁-C₄-Alkylammoniumsalzen.

5. Katalysatorzusammensetzung nach einem der vorhergehenden Ansprüche, **dadurch gekennzeichnet, daß** die Stickstoffquelle ausgewählt ist unter wasserlöslichen Ammonium- und C₁-C₄-Alkylammoniumsalzen oder einem wasserlöslichen Nitratsalz des eingesetzten Nichtedelmetalls, und daß die Phosphatkomponente Dihydrogenphosphationen sind.

6. Katalysatorzusammensetzung nach einem der vorhergehenden Ansprüche, **dadurch gekennzeichnet, daß** das Nichtedelmetallsalz ausgewählt ist unter Salzen, die Eisen in der Oxidationsstufe +2, +3, +4, +5 und/oder +6 enthalten, und Salzen, die Zinn in der Oxidationsstufe +2 und/oder +4 enthalten.

7. Katalysatorzusammensetzung nach einem der vorhergehenden Ansprüche, dadurch erhältlich, daß man das aus Stufe a) erhaltene wäßrige Gemisch bei einem Druck von etwa 15 bis etwa 1000 mbar und bei einer Temperatur im Bereich von etwa 10 bis etwa 200°C einengt und den so erhaltenen Rückstand bei einer Temperatur von etwa 30 bis 200°C trocknet.

8. Katalysatorzusammensetzung nach einem der vorhergehenden Ansprüche, **dadurch gekennzeichnet, daß** sie als Nichtedelmetallkomponente Eisenionen enthält und ein Röntgendiffraktogramm zeigt, das folgende Beugungslinien umfaßt:
| 2-Theta | d |
|---|---|
| 9,37 | 9,429 |
| 18,37 | 4,824 |
| 28,01 | 3,183 |
| 28,78 | 3,099 |
| 35,05 | 2,558 |
| 37,87 | 2,373 |
oder daß sie als Nichtedelmetallkomponente zinnionen enthält und ein Röntgendiffraktogramm zeigt, das folgende Beugungslinien umfaßt:
| 2-Theta | d |
|---|---|
| 12,79 | 6,915 |
| 13,04 | 6,784 |
| 19,09 | 4,645 |
| 20,21 | 4,389 |
| 23,01 | 3,861 |
| 23,90 | 3,720 |
| 26,18 | 3,400 |
| 30,33 | 2,944 |

9. Katalysatorzusammensetzung, enthaltend eine edelmetallfreie Katalysatorkomponente nach einem der vorhergehenden Ansprüche sowie als weitere katalytisch aktive Komponente einen Sauerstoffüberträger.

10. Katalysatorzusammensetzung nach Anspruch 9, **dadurch gekennzeichnet, daß** der Sauerstoffüberträger ausgewählt ist unter metallorganischen Verbindungen, Zeolithen, zeolithanalogen Alumophosphaten oder mesoporösen Metalloxiden, welche jeweils mindestens ein Metall, ausgewählt unter Ti, V, Mo, W, Re und Ru, enthalten.

11. Katalysatorzusammensetzung nach Anspruch 10, **dadurch gekennzeichnet, daß** der Sauerstoffüberträger ein Titan- oder Vanadiumsilikat mit Pentasilstruktur ist.

12. Katalysatorzusammensetzung nach einem der Ansprüche 1 bis 11 auf einem festen, inerten Träger.

13. Verfahren zur Herstellung von Wasserstoffperoxid, **dadurch gekennzeichnet, daß** man Wasserstoff und Sauerstoff in Gegenwart einer Katalysatorzusammensetzung nach einem der Ansprüche 1 bis 8 umsetzt.

14. Verfahren zur Epoxidation von Olefinen, **dadurch gekennzeichnet, daß** man das Olefin in Gegenwart einer Katalysatorzusammensetzung gemäß einem der Ansprüche 9 bis 12 mit Wasserstoff und Sauerstoff umsetzt.

## Claims

1. A noble metal-free catalyst composition obtainable by
a) preparing an aqueous mixture comprising
i) a salt of at least one base metal selected from among the elements having atomic numbers 21-32, 39-42, 48-51, 57-75 and 81-83;
ii) phosphate ions; and
iii) at least one nitrogen source; and
b) evaporating the aqueous mixture obtained and drying the catalyst composition thus formed, where base metal (M), phosphate (P) and nitrogen (N) are present in the catalytically active, dried solid in a molar ratio of M : P : N = 1 : 0.9-1.3 : 0.9-1.7.

2. A catalyst composition as claimed in claim 1, wherein the aqueous solution comprises metal ions (M), phosphate ions (P) and a nitrogen source (N) in a molar ratio of M : P : N = 1 : 0.8-1.4 : 0.6-4.0.

3. A catalyst composition as claimed in either of the preceding claims, wherein the base metal salt is selected from among water-soluble salts of metals having atomic numbers 21-32, 39-42 and 48-51.

4. A catalyst composition as claimed in any of the preceding claims, wherein the nitrogen source is selected from among nitric acid and the noble metal-free, water-soluble salts thereof, ammonium, amines, ammonium or C₁-C₄-alkylammonium salts.

5. A catalyst composition as claimed in any of the preceding claims, wherein the nitrogen source is selected from among water-soluble ammonium and C₁-C₄-alkylammonium salts or a water-soluble nitrate salt of the base metal used, and the phosphate component comprises dihydrogenphosphate ions.

6. A catalyst composition as claimed in any of the preceding claims, wherein the base metal salt is selected from among salts containing iron in the oxidation state +2, +3, +4, +5 and/or +6 and salts containing tin in the oxidation state +2 and/or +4.

7. A catalyst composition as claimed in any of the preceding claims, obtainable by evaporating the aqueous mixture obtained from stage a) at a pressure of from about 15 to about 1000 mbar and at from about 10 to about 200°C and drying the residue thus obtained at from about 30 to 200°C.

8. A catalyst composition as claimed in any of the preceding claims, wherein the base metal component present comprises iron ions and the composition displays an X-ray diffractogram comprising the following diffraction lines:
| 2-theta | d |
|---|---|
| 9.37 | 9.429 |
| 18.37 | 4.824 |
| 28.01 | 3.183 |
| 28.78 | 3.099 |
| 35.05 | 2.558 |
| 37.87 | 2.373 |
or wherein the base metal component present comprises tin ions and the composition displays an X-ray diffractogram comprising the following diffraction lines:
| 2-theta | d |
|---|---|
| 12.79 | 6.915 |
| 13.04 | 6.784 |
| 19.09 | 4.645 |
| 20.21 | 4.389 |
| 23.01 | 3.861 |
| 23.90 | 3.720 |
| 26.18 | 3.400 |
| 30.33 | 2.944 |

9. A catalyst composition comprising a noble metal-free catalyst component as claimed in any of the preceding claims and also an oxygen transferer as further catalytically active component.

10. A catalyst composition as claimed in claim 9, wherein the oxygen transferer is selected from among organometallic compounds, zeolites, zeolite-analogous aluminophosphates or mesoporous metal oxides which each comprise at least one metal selected from among Ti, V, Mo, W , Re and Ru.

11. A catalyst composition as claimed in claim 10, wherein the oxygen transferer is a titanium or vanadium silicate having a pentasil structure.

12. A catalyst composition as claimed in any of claims 1 to 11 on a solid, inert support.

13. A process for preparing hydrogen peroxide, which comprises reacting hydrogen and oxygen in the presence of a catalyst composition as claimed in any of claims 1 to 8.

14. A process for the epoxidation of olefins, which comprises reacting the olefin with hydrogen and oxygen in the presence of a catalyst composition as claimed in any of claims 9 to 12.

## Revendications

1. Composition de catalyseur exempte de métaux précieux que l'on peut obtenir
a) en fabriquant un mélange aqueux qui contient
i) un sel d'au moins un métal non précieux choisi parmi les éléments ayant un nombre atomique compris entre 21 et 32, 39 et 42, 48 et 51, 57 et 75 ou 81 et 83;
ii) des ions phosphate; et
iii) au moins une source d'azote; et
b) en concentrant le mélange aqueux ainsi obtenu et en séchant la masse de catalyseur ainsi produite,
dans laquelle, dans la matière séchée à activité catalytique, le rapport molaire M:P:N entre le métal non précieux (M), le phosphate (P) et l'azote (N) est compris dans la plage de 1 : 0,9 à 1,3 : 0,9 à 1,7.

2. Composition de catalyseur selon la revendication 1, **caractérisée en ce que** la solution aqueuse contient les ions de métal (M), les ions de phosphate (P) et la source d'azote (N) dans un rapport molaire M:P:N = 1 : 0,8 à 1,4 : 0,6 à 4,0.

3. Composition de catalyseur selon l'une quelconque des revendications précédentes, **caractérisée en ce que** l'on choisit le sel de métal non précieux parmi les sels solubles dans l'eau et dérivés d'un métal ayant un nombre atomique compris entre 21 et 32, 39 et 42 ou 48 et 51.

4. Composition de catalyseur selon l'une quelconque des revendications précédentes, **caractérisée en ce que** l'on choisit la source d'azote dans le groupe formé par l'acide nitrique et ses sels solubles dans l'eau et exempts de métaux précieux, l'ammoniac, les amines, les sels d'ammonium et de (alkyle en C₁ à C₄)-ammonium.

5. Composition de catalyseur selon l'une quelconque des revendications précédentes, **caractérisée en ce que** l'on choisit la source d'azote dans le groupe formé par les sels d'ammonium et de (alkyle en C₁ à C₄)-ammonium solubles dans l'eau et les sels de nitrate solubles dans l'eau et dérivés du métal non précieux que l'on a mis auparavant en oeuvre, et **caractérisée en ce que** le composant phosphate est constitué d'ions dihydrogénophosphates.

6. Composition de catalyseur selon l'une quelconque des revendications précédentes, **caractérisée en ce que** l'on choisit le sel de métal non précieux dans le groupe formé par les sels contenant le fer présentant un degré d'oxydation +2, +3, +4, +5 et/ou +6 et les sels contenant l'étain présentant un degré d'oxydation +2 et/ou +4.

7. Composition de catalyseur selon l'une quelconque des revendications précédentes, que l'on peut obtenir en concentrant, à une pression comprise entre environ 15 mbars et environ 1 000 mbars et à une température comprise dans la plage d'environ 10°C à environ 200°C, le mélange aqueux obtenu dans l'étape a), suivi d'un séchage du résidu ainsi obtenu à une température comprise entre environ 30°C et 200°C.

8. Composition de catalyseur selon l'une quelconque des revendications précédentes, **caractérisée en ce qu'**elle contient des ions de fer, en tant que composant métallique non précieux, et présente un diagramme de diffraction comprenant les raies de diffraction suivantes :
| 2-thêta | d |
|---|---|
| 9,37 | 9,429 |
| 18,37 | 4,824 |
| 28,01 | 3,183 |
| 28,78 | 3,099 |
| 35,05 | 2,558 |
| 37,87 | 2,373 |
ou **caractérisée en ce qu'**elle contient les ions d'étain, en tant que composant métallique non précieux, et présente un diagramme de diffraction comprenant les raies de diffraction suivantes :
| 2-thêta | d |
|---|---|
| 12,79 | 6,915 |
| 13,04 | 6,784 |
| 19,09 | 4,645 |
| 20,21 | 4,389 |
| 23,01 | 3,861 |
| 23,90 | 3,720 |
| 26,18 | 3,400 |
| 30,33 | 2,944 |

9. Composition de catalyseur contenant un composant catalyseur exempt de métaux précieux selon l'une quelconque des revendications précédentes ainsi que, en tant que composant à activité catalytique supplémentaire, un vecteur d'oxygène.

10. Composition de catalyseur selon la revendication 9, **caractérisée en ce que** l'on choisit le vecteur d'oxygène dans le groupe formé par les composés organométalliques, les zéolithes, les aluminophosphates analogues de zéolithe et les oxydes métalliques mésoporeux contenant au moins un atome de métal, chacun étant choisi dans le groupe formé par les atomes de Ti, V, Mo, W, Re et Ru.

11. Composition de catalyseur selon la revendication 10, **caractérisée en ce que** le vecteur d'oxygène est un silicate de titane ou de vanadium présentant la structure de pentasil.

12. Composition de catalyseur selon l'une quelconque des revendications 1 à 11 sur un support solide et inerte.

13. Procédé de fabrication du peroxyde d'hydrogène, **caractérisé en ce que** l'on met à réagir de l'hydrogène et de l'oxygène en présence d'une composition de catalyseur selon l'une quelconque des revendications 1 à 8.

14. Procédé de époxydation d'oléfines, **caractérisé en ce que** l'on met à réagir une oléfine, de l'hydrogène et de l'oxygène en présence d'une composition de catalyseur selon l'une quelconque des revendications 9 à 12.
